# EUROPÄISCHE PATENTANMELDUNG

(11) **EP 2 503 481 A1**
(43) Veröffentlichungstag der Anmeldung: **26.09.2012**
(21) Anmeldenummer: 11159626.8
(22) Anmeldetag: 24.03.2011
(51) Int. Cl.: G06F 21/00, G06F 21/24, G06F 19/00

(54) **Blutzuckermessgerät und Verfahren zum Auslesen von Blutzuckermesswerten**

(71) Anmelder: F. Hoffmann-La Roche AG, 4070 Basel (CH); Roche Diagnostics GmbH, 68305 Mannheim (DE)
(72) Erfinder: Porsch, Ulrich, 69469 Weinheim (DE); Werner, Karl, 69168 Wiesloch (DE); Riebel, Stefan, 68161 Mannheim (DE)
(74) Vertreter: Bittner, Thomas L.

(57) **Zusammenfassung**

Die Erfindung betrifft ein Blutzuckermessgerät (1), mit einer Messeinrichtung (2) zum Bestimmen von Blutzuckermesswerten, einem Messwertspeicher (5), in welchem die Blutzuckermesswerte gespeichert sind, einer Datenschnittstelle (6), die für einen Datenaustausch mit einer externen Datenauslesevorrichtung (7) konfiguriert ist, und einem Datenfilesystem (10), welches eine Datenauslese-Applikation umfasst, auf die die externe Datenauslesevorrichtung (7) für ein Auslesen der gespeicherten Blutzuckermesswerte zugreifen kann, wobei das Datenfilesystem (10) weiterhin eine Prüfapplikation umfasst, die konfiguriert ist, eine Integritätsprüfung für die Datenauslese-Applikation auszuführen, indem eine aktuelle digitale Signatur für die Datenauslese-Applikation gebildet und mit einer in dem Datenfilesystem (10) bereitgestellten digitalen Prüfsignatur verglichen wird. Des Weiteren betrifft die Erfindung ein Verfahren zum Auslesen von Blutzuckermesswerten von einem Blutzuckermessgerät (1).

## Beschreibung

Die Erfindung betrifft ein Blutzuckermessgerät sowie ein Verfahren zum Auslesen von Blutzuckermesswerten von einem Blutzuckermessgerät.

### Hintergrund der Erfindung

Blutzuckermessgeräte werden benutzt, um Messwerte für den Blutzuckerwert zu bestimmen. Die hierbei erfassten Blutzuckermesswerte werden in Form elektronischer Daten in einer Speichereinrichtung des Blutzuckermessgerätes gespeichert. Sie stehen auf diese Weise für beliebige anschließende Auswertungen der Messergebnisse zur Verfügung. Eine solche Auswertung kann auf dem Blutzuckermessgerät selbst und / oder auch außerhalb des Messgerätes erfolgen, indem die Blutzuckermesserwerte über eine Datenschnittstelle des Blutzuckermessgerätes mit Hilfe einer externen Datenauslesevorrichtung ausgelesen werden. Bei der Auslesevorrichtung handelt es sich beispielsweise um einen Personalcomputer, auf dem Software-applikationen implementiert sein können, um die Blutzuckermesswerte zum Beispiel grafisch darzustellen oder beliebige andere Auswertungen durchzuführen.

Im Zusammenhang mit dem Auslesen oder Herunterladen der Blutzuckermesswerte wurde vorgeschlagen, den Zugriff auf die auszulesenden Blutzuckermesswerte mit Hilfe eines auf dem Personalcomputer implementierten Browsers auszuführen, wie er beispielsweise auch für den Zugriff auf Internetseiten nutzbar ist. Die Verwendung des Browsers ermöglicht das Auslesen der Blutzuckermesswerte, ohne dass der Personalcomputer mit einer speziellen Auslese-Software versehen wird. Das Auslesen oder Herunterladen der Blutzuckermesswerte kann mit Hilfe eines üblichen Browsers erfolgen. Zum Auslesen der Blutzuckermesswerte wird hierbei von dem Browser eine dem Auslesevorgang zugeordnete Applikation im Blutzuckermessgerät selbst gestartet. Im Rahmen dieser Applikation werden dann die Messwerte von dem Blutzuckermessgerät auf das Auslesegerät übertragen.

Da die Auslesevorrichtung hierbei nicht mit einer speziellen Software zum Auslesen oder Herunterladen der Blutzuckermesswerte ausgestattet ist, besteht das Risiko, dass Probleme oder Fehler bei der Datenübermittlung der Blutzuckermesswerte auftreten, zum Beispiel wegen nicht korrekter Kompatibilität von Datenformaten. Dieses kann beispielsweise dazu führen, dass die Darstellung der Messwerte in grafischen Darstellungen verfälscht ist, wodurch das Risiko einer Fehldiagnose besteht.

### Zusammenfassung der Erfindung

Aufgabe der Erfindung ist es, ein Blutzuckermessgerät sowie ein Verfahren zum Auslesen von Blutzuckermesswerten von einem Blutzuckermessgerät anzugeben, mit denen die Sicherheit für eine korrekte Datenübermittlung der Blutzuckermesswerte auf eine externe Datenauslesevorrichtung verbessert ist.

Diese Aufgabe wird erfindungsgemäß durch ein Blutzuckermessgerät nach dem unabhängigen Anspruch 1 gelöst. Weiterhin ist ein Verfahren zum Auslesen von Blutzuckermesswerten von einem Blutzuckermessgerät nach dem unabhängigen Anspruch 8 geschaffen. Vorteilhafte Ausgestaltungen der Erfindung sind Gegenstand von abhängigen Unteransprüchen.

Die Erfindung umfasst den Gedanken eines Blutzuckermessgerätes, mit einer Messeinrichtung zum Bestimmen von Blutzuckermesswerten, einem Messwertspeicher, in welchem Blutzuckermesswerte gespeichert sind, einer Datenschnittstelle, die für einen Datenaustausch mit einer externen Datenauslesevorrichtung konfiguriert ist, und einem Datenfilesystem, welches eine Datenauslese-Applikation umfasst, auf die die externe Datenauslesevorrichtung für ein Auslesen der gespeicherten Blutzuckermesswerte zugreift, wobei das Datenfilesystem weiterhin eine Prüfapplikation umfasst, die konfiguriert ist, eine Integritätsprüfung für die Datenauslese-Applikation auszuführen, indem eine aktuelle digitale Signatur für die Datenauslese-Applikation gebildet und mit einer in dem Datenfilesystem bereitgestellten digitalen Prüfsignatur verglichen wird.

Nach einem weiteren Aspekt ist ein Verfahren zum Auslesen von Blutzuckermesswerten von einem Blutzuckermessgerät geschaffen, wobei das Verfahren die folgenden Schritte aufweist:
- Ausführen einer in einem Datenfilesystem des Blutzuckermessgerätes gebildeten Prüfapplikation, bei der eine Integritätsprüfung für eine ebenfalls in dem Datenfilesystem gebildete Datenauslese-Applikation ausgeführt wird, derart, dass eine aktuelle digitale Signatur für die Datenauslese-Applikation gebildet und mit einer in dem Datenfilesystem bereitgestellten digitalen Prüfsignatur verglichen wird, und
- Unterbinden einer Übertragung von Blutzuckermesswerten von dem Blutzuckermessgerät auf die externe Datenauslesevorrichtung im Rahmen der Datenauslese-Applikation und / oder einer Anzeige übertragener Blutzuckermesswerte auf einer externen Anzeigeeinrichtung, wenn die Prüfapplikation ergibt, dass die aktuelle digitale Signatur und die digitale Prüfsignatur in unzulässiger Weise nicht übereinstimmen.

Mit Hilfe der Erfindung wird sichergestellt, dass das Ausführen einer Datenauslese-Applikation unter Verwendung einer korrekten und intakten Version der Datenauslese-Applikation ausgeführt wird. Bevor das tatsächliche Herunterladen von Blutzuckermesswerten von dem Blutzuckermessgerät auf die externe Auslesevorrichtung, bei der es sich beispielsweise um einen Personalcomputer handelt, beginnen kann, wird eine Integritäts- oder Sicherheitsprüfung für die Datenauslese-Applikation durchgeführt. Hierbei wird eine aktuelle digitale Signatur für die Datenauslese-Applikation gebildet und mit einer in dem Blutzuckermessgerät hinterlegten und für die Prüfapplikation in dem Datenfilesystem bereitgestellten digitalen Prüfsignatur verglichen.

In einer Ausführungsform wird danach der Zugriff auf die Blutzuckermesswerte zum Herunterladen nur dann freigegeben, wenn die Integritätsprüfung ergibt, dass die Datenauslese-Applikation intakt ist. Andernfalls kann der Zugriff auf die Blutzuckermesswerte, also eine tatsächliche weitere Ausführung der Datenauslese-Applikation in Form des Herunterladens der Blutzuckermesswerte gesperrt werden.

In einer weiteren Ausgestaltung kann vorgesehen sein, dass die Ausführung der Datenauslese-Applikation nicht nur das (bloße) Herunterladen oder Abrufen der Blutzuckermesswerte bewirkt, sondern ergänzend eine Darstellung der herunter geladenen Blutzuckermesswerte, also ein Anzeigen auf einer elektronischen Anzeigeeinrichtung, die von der externen Datenauslesevorrichtung umfasst ist oder an diese koppelt. Das Anzeigen kann im Rahmen einer Anzeigeapplikation ausgeführt werden, die Teil der Datenausleseapplikation oder getrennt hiervon gebildet ist, zum Beispiel als Unterapplikation, wobei sie insbesondere dann vorzugsweise automatisch durch die Datenauslese-Applikation aufrufbar ist. Die Anzeige kann zeitgleich mit dem Herunterladen und / oder zeitverzögert hierzu erfolgen. Bei dieser Ausführungsform kann dann ebenfalls vorgesehen sein, den Zugriff auf die Blutzuckermesswerte zum tatsächlichen Abrufen im Rahmen der Datenauslese-Applikation zu sperren. Alternativ kann vorgesehen sein, dass bei fehlender Integrität der Datenauslese-Applikation zwar ein Herunterladen der Messwerte noch möglich ist, insbesondere in Form von Rohdaten, die zur grafischen Darstellung noch aufzubereiten sind, aber das anschließende Anzeigen der herunter geladenen Daten unterbunden ist. So wird beispielsweise verhindert, dass eventuell fehlerhaft herunter geladene Blutzuckermesswerte zur Anzeige kommen.

Die aktuelle digitale Signatur wird für den Software- oder Programmcode der Datenauslese-Applikation selbst gebildet. Es handelt sich also um eine Integritätsprüfung für den Software-oder Programmcode der Datenauslese-Applikation.

Es kann vorgesehen sein, die Prüfapplikation (erst) bei einem Zugriff der externen Datenauslesevorrichtung auf die Datenauslese-Applikation automatisch zu starten. Die Prüfapplikation ist bei dieser Ausgestaltung konfiguriert, - ausgelöst durch das Zugreifen der externen Datenauslösevorrichtung auf die Datenauslese-Applikation - eine Integritätsprüfung für die Datenauslese-Applikation auszuführen, indem die aktuelle digitale Signatur für die Datenauslese-Applikation gebildet und mit der in dem Datenfilesystem bereitgestellten digitalen Prüfsignatur verglichen wird. Alternaiv oder ergänzend kann vorgesehen sein, eine Integritätsprüfung für die Datenauslese-Applikation proaktiv einmalig oder mehrmalig durchzuführen, zum Beispiel bei einem Anschalten des Blutzuckermessgerätes oder ausgelöst durch ein anderes Ereignis wie einer Firmware-Änderung, ohne dass zu diesem Zeitpunkt ein Zugriff auf die Datenauslese-Applikation durch die externe Datenauslesevorrichtung tatsächlich gegeben ist. Den vorangehend beschriebenen Ausgestaltungsformen entsprechend wird jeweils eine aktuelle digitale Signatur zum Vergleich mit der digitalen Prüfsignatur bereitgestellt. Der Begriff "aktuell" im Sinne der hier verwendeten Bedeutung besagt, dass es sich um die digitale Signatur für die zum Zeitpunkt des Ausführens der Prüfapplikation vorliegende Version der Datenauslese-Applikation handelt, wobei diese in korrekter oder nicht korrekter Form vorliegen kann, wobei Letzteres dann zu den oben beschriebenen Folgen führt.

Das Zugreifen der externen Datenauslesevorrichtung auf die Datenauslese-Applikation erfolgt beispielsweise mit einer auf der externen Auslesevorrichtung implementieren Browsereinrichtung. Hierbei handelt es sich um eine Applikation auf der externen Datenauslesevorrichtung, die üblicherweise nicht speziell für das Verfahren zum Auslesen und zum wahlweisen Anzeigen der Blutzuckermesswerte hergerichtet ist. Vielmehr kann die Browsereinrichtung flexibel in verschiedenen Anwendungen eingesetzt werden, zum Beispiel beim Zugriff auf Internetseiten. Die Browsereinrichtung greift auf die Datenauslese-Applikation im Datenfilesystem des Blutzuckermessgerätes zu, um einen Datenauslesevorgang und eine wahlweise sich anschließende Messwertdarstellung zu starten. Erst wenn die Prüfapplikation ergibt, dass die Datenauslese-Applikation noch in korrekter Form vorliegt, können anschließend ein Herunterladen und ein eventuell vorgesehenes Anzeigen der Blutzuckermesswerte tatsächlich stattfinden. Hierdurch wird ein korrektes und sicheres Übermitteln der Blutzuckermesswerte von dem Blutzuckermessgerät an die externe Datenauslesevorrichtung unterstützt. Die Datenübermittlung selbst kann wahlweise mit weiteren Sicherungsmechanismen ausgeführt werden, beispielsweise unter Verwendung einer Verschlüsselung der zu übertragenden Messwerte.

Für die Integritätsprüfung der Datenauslese-Applikation wird die digitale Signatur verwendet. Hierbei handelt es sich um ein kryptografisches Verfahren, bei dem für den Software- oder Programmcode der Datenauslese-Applikation ein Zahlenwert berechnet wird. Digitale Signaturverfahren sind in verschiedenen Ausführungsformen als solche bekannt. Hierzu gehört beispielsweise das sogenannte RSA oder DSA. Häufig erfolgt die Berechnung der Signatur unter Einbeziehung eines sogenannten Hash-Wertes. Eine einfache Form der digitalen Signaturprüfung in der hier verendeten Bedeutung ist die sogenannte Checksummenprüfung.

Eine bevorzugte Weiterbildung der Erfindung sieht vor, dass durch einen nichtflüchtigen Signaturspeicher, in welchem die digitale Prüfsignatur gespeichert ist. Der nichtflüchtige Signaturspeicher ist beispielsweise als ein elektronisch löschbarer programmierbarer Speicher ausgeführt (EEPROM).

Bei einer zweckmäßigen Ausgestaltung der Erfindung kann vorgesehen sein, dass durch ein Firmwaremodul, welches konfiguriert ist, die digitale Prüfsignatur aus dem nichtflüchtigen Signaturspeicher auszulesen und an das Datenfilesystem zu übergeben. Das Bereitstellen der digitalen Prüfsignatur in dem Datenfilesystem kann erfolgen, bevor ein tatsächlicher Zugriff einer externen Auslesevorrichtung auf die Datenauslese-Applikation erfolgt. Insoweit ist das Blutzuckermessgerät dann bereits proaktiv für einen solchen Zugriff eingerichtet. Es kann aber auch vorgesehen sein, die digitale Prüfsignatur in Echtzeit beim Feststellen eines Zugriffs der externen Datenauslesevorrichtung aus dem Signaturspeicher in das Datenfilesystem zu übertragen. Auch kann in einer Ausgestaltung vorgesehen sein, auf diese Weise eine Aktualitätsprüfung für die in dem Datenfilesystem gespeicherte digitale Prüfsignatur auszuführen. Wenn also ein Zugriff auf die Datenauslese-Applikation festgestellt wird, erfolgt in einem Schritt zur Aktualitätsprüfung ein Vergleich der aktuell aus dem Signaturspeicher ausgelesenen digitalen Prüfsignatur mit einer bereits im Datenfilesystem vorhandenen digitalen Prüf-signatur. Auch kann vorgesehen sein, eine solche Aktualitätsprüfung losgelöst von einem tatsächlichen Zugriff auf die Datenauslese-Applikation wiederholt durchzuführen, zum Beispiel in vorgegebenen Zeitabständen.

Eine vorteilhafte Ausführungsform der Erfindung sieht vor, dass die Prüfapplikation als eine Unterapplikation der Datenauslese-Applikation gebildet ist, derart, dass de Unterapplikation nach einem Start der Datenauslese-Applikation durch diese gestartet wird. Bei dieser Ausgestaltung ruft die Datenauslese-Applikation die Prüfapplikation selbst auf. In einer alternativen Ausführungsform kann vorgesehen sein, dass bei einem festgestellten Zugriffsversuch auf die Datenauslese-Applikation zunächst die Prüfapplikation aufgerufen und durchgeführt wird, bevor dann eine Ausführung der Datenauslese-Applikation selbst tatsächlich gestartet wird.

Bevorzugt sieht eine Fortbildung der Erfindung vor, dass die Datenauslese-Applikation und die Prüfapplikation jeweils als eine Skriptapplikation gebildet sind. Beispielsweise können die Applikationen als Javascript-Applikationen gebildet sein. Scriptapplikationen können von einer auf der externen Datenauslesevorrichtung implementierten Browsereinrichtung beliebiger Art aufgerufen und gestartet werden.

Bei einer vorteilhaften Ausgestaltung der Erfindung kann vorgesehen sein, dass die digitale Prüfsignatur in dem Datenfilesystem in einer Konfigurationsdatei bereitgestellt ist. Bei dieser Ausführungsform ist die digitale Prüfsignatur in eine Konfigurationsdatei integriert, die wahlweise weitere elektronische Daten umfassen kann, die insbesondere Parameter für den Betrieb des Blutzuckermessgerätes festlegen. Zu solchen Betriebsparametern gehören beispielsweise die Maßeinheit, in welcher die Blutzuckremesswerte angezeigt werden, und die Sprache, welche in den angezeigten Grafiken verwendet wird.

Eine Weiterbildung der Erfindung kann vorsehen, dass die Datenauslese-Applikation konfiguriert ist, für einzelne Blutzuckermesswerte und / oder eine Gruppen von Blutzuckermesswerten eine Messwert-Integritätsprüfung auszuführen, indem eine digitale Messwert-Signatur gebildet und mit einer digitalen Messwert-Prüfsignatur verglichen wird. Bei dieser Ausführungsform werden die vorgesehenen Sicherheitsmechanismen für die Datenübermittlung beim Auslesen der Blutzuckermesswerte ergänzt durch eine Integritätsprüfung für die Messwerte selbst. Auch die digitale Messwert-Prüfsignatur kann in dem Signaturspeicher abgelegt sein. Die Handhabung der digitalen Messwert-Prüfsignatur kann in verschiedenen Ausführungsformen den obigen Erläuterungen für die digitale Prüfsignatur entsprechend erfolgen. Beabsichtigte oder unbeabsichtigte Messwertverfälschungen können so unterbunden werden.

Bei einer Fortbildung der Erfindung kann vorgesehen sein, dass die Datenauslese-Applikation und / oder die Prüfapplikation konfiguriert sind, ein Herunterladen der Blutzuckermesswerte auf die Datenauslesevorrichtung und / oder ein Anzeigen herunter geladener Blutzuckermesswerte zu unterbinden, wenn die Prüfapplikation ergibt, dass aktuelle digitale Signatur und die digitale Prüfsignatur in unzulässiger Weise nicht übereinstimmen. Eine fehlende Übereinstimmung ist insbesondere dann gegeben, wenn die beiden Signaturen nicht denselben Wert aufweisen.

In einer Ausgestaltung kann das Verfahren die folgenden Schritte umfassen:
- Zugreifen einer externen Datenauslesevorrichtung über eine Datenschnittstelle auf eine Datenauslese-Applikation in einem Datenfilesystem eines Blutzuckermessgerätes,
- Ausführen einer in dem Datenfilesystem des Blutzuckermessgerätes gebildeten Prüfapplikation, bei der eine Integritätsprüfung für die Datenauslese-Applikation ausgeführt wird, derart, dass eine aktuelle digitale Signatur für die Datenauslese-Applikation gebildet und mit einer in dem Datenfilesystem bereitgestellten digitalen Prüfsignatur verglichen wird,
- Freigeben der Ausführung der Datenauslese-Applikation, wenn bei der Prüfapplikation festgestellt wird, dass die aktuelle digitale Signatur für die Datenauslese-Applikation der digitalen Prüfsignatur entspricht, und
- Übertragen von Blutzuckermesswerten von dem Blutzuckermessgerät auf die externe Datenauslesevorrichtung im Rahmen der Datenauslese-Applikation.

### Beschreibung bevorzugter Ausführungsbeispiele der Erfindung

Die Erfindung wird im Folgenden anhand von bevorzugten Ausführungsbeispielen unter Bezugnahme auf eine Figur näher erläutert.

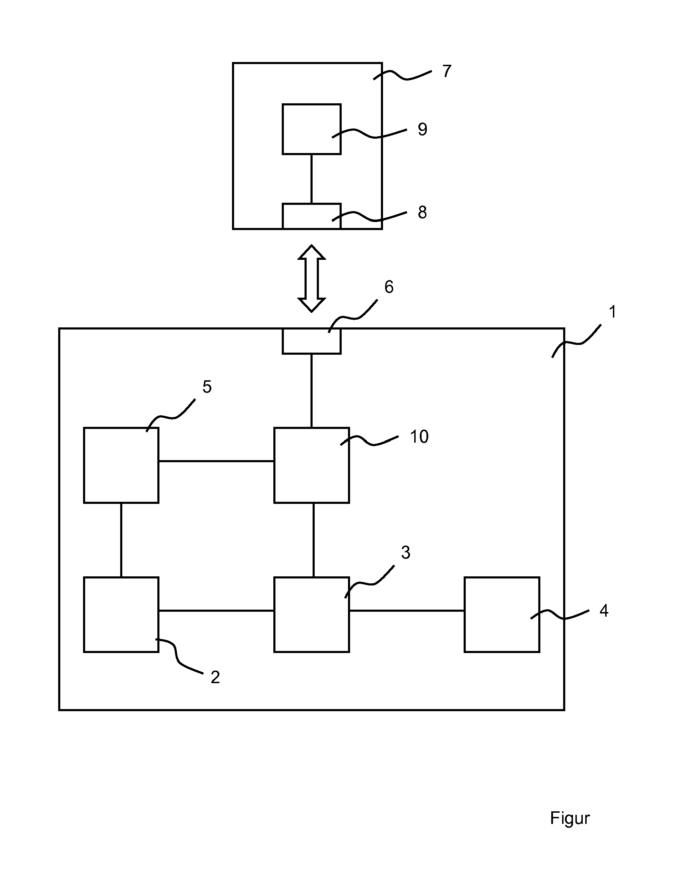

Die einzige Figur zeigt eine schematische Darstellung eines Systems zum Erfassen und Auswerten von Blutzuckermesswerten. Das System weist ein Blutzuckermessgerät 1 auf, welches mit Hilfe einer Blutzuckermesseinrichtung 2 konfiguriert ist, Blutzuckermesswerte für Blutproben zu erfassen, sei es in einer kontinuierlichen und / oder diskontinuierlichen Betriebsart. Blutzuckermesseinrichtungen sind als solche in verschiedenen Ausführungsformen bekannt, weshalb dieses hier nicht weiter zu erläutern ist.

Ein Firmwaremodul 3 umfasst in zugeordnete Speicherelemente eingebetteten ("embedded") Softwarecode. Hierbei handelt es sich dem üblichen Verständnis entsprechend um gerade nicht von Anwendungssoftware erfasste Komponenten, die funktionell an die Hardware des Blutzuckermessgerätes 1 gebunden sind. Der im Firmwaremodul 3 enthaltene Softwarecode ist vom Benutzer, also insbesondere einem Patienten oder medizinischem Personal, nicht beeinflussbar.

Das Firmwaremodul 3 ist konfiguriert, auf einen Signaturspeicher 4 in dem Blutzuckermessgerät 1 zuzugreifen. Der Signaturspeicher 4 ist ein nichtflüchtiger Speicher, beispielsweise ein EEPROM, in welchem digitale Signaturen gespeichert werden.

Die mit Hilfe der Blutzuckermesseinrichtung 2 erfassten Blutzuckermesswerte werden in einem Messwertspeicher 5 gespeichert. Dieses erfolgt bevorzugt mittels sogenannter "embedded" Nachrichten (Reports). Die in dem Messwertspeicher 5 gespeicherten Informationen über Blutzuckermesswerte sind über eine Datenschnittstelle 6 des Blutzuckermessgerätes 1 mit Hilfe einer externen Datenauslesevorrichtung 7 herunterlad- oder auslesbar. Bei der externen Datenauslesevorrichtung 7 handelt es sich beispielsweise um einen Personalcomputer. Die Datenübertragung zwischen der Datenschnittstelle 6 des Blutzuckermessgerätes 1 und einer von der externen Datenauslesevorrichtung 7 umfassten Datenschnittstelle 8 erfolgt über eine kabelgebundene oder drahtlose Kommunikationsverbindung, wahlweise unter Einbeziehung des Internets und / oder anderer Datenkommunikationsnetzwerke.

Auf der externen Datenauslesevorrichtung 7 ist eine Browsereinrichtung 9 implementiert, mit der über die Datenschnittstellen 6, 8 auf ein Datenfilesystem 10 in dem Blutzuckermessgerät 1 zugegriffen werden kann. Das Datenfilesystem 10 umfasst verschiedene elektronische Daten und Informationen. Hierzu gehören insbesondere ausführbare Software-Applikationen, die zum Beispiel als Scriptapplikationen vorliegen, bevorzugt als Javascript-Applikationen. Aber auch für das Auslesen oder das Herunterladen bereitgestellte Messwerte können in dem Datenfilesystem 10 abgelegt sein. Darüber hinaus sind in dem Datenfilesystem 10 ein oder mehrere digitale Prüfsignaturen bereitgestellt, die vorzugsweise mittels des Firmwaremoduls 3 aus dem Signaturspeicher 4 in das Datenfilesystem 10 übertragen wurden.

Sollen nun in dem beschriebenen System Blutzuckermesswerte von dem Blutzuckermessgerät 1 auf die externe Datenauslesevorrichtung 7 heruntergeladen werden, greift die Browsereinrichtung 9 über die Datenschnittstellen 6, 8 auf eine Datenauslese-Applikation in dem Datenfilesystem 10 zu. Wird ein solcher Zugriff auf die Datenauslese-Applikation in dem Blutzuckermessgerät 1 festgestellt, wird eine weitere in dem Datenfilesystem 10 vorhandene Applikation gestartet, nämlich eine Prüfapplikation. Im Rahmen der ausgeführten Prüfapplikation wird für den die Datenauslese-Applikation bildenden Softwarecode eine Integritätsprüfung durchgeführt, indem für den Softwarecode eine aktuelle digitale Signatur ermittelt und mit einer der Datenauslese-Applikation zugeordneten digitalen Prüfsignatur verglichen wird. Wenn eine Übereinstimmung der digitalen Signaturen festgestellt wird, kann anschließend das Herunterladen oder Auslesen der Blutzuckermesswerte mittels der Datenauslese-Applikation ausgeführt werden. Andernfalls wird eine Ausführung der Datenauslese-Applikation gesperrt.

Die für die Prüfapplikation herangezogene digitale Prüfsignatur für die Datenauslese-Applikation war zuvor in dem Signaturspeicher 4 hinterlegt. Es kann vorgesehen sein, die digitale Prüfsignatur bereits vor einem Zugriff auf die Datenauslese-Applikation in dem Datenfilesystem 10 proaktiv zu hinterlegen. Aber auch ein Echtzeitzugriff auf die digitale Prüfsignatur beim Feststellen eines (versuchten) Zugriffs auf die Datenauslese-Applikation durch die Browsereinrichtung 9 kann vorgesehen sein. In dem Datenfilesystem 10 kann die digitale Prüfsignatur in eine Konfigurationsdatei integriert sein.

In einer alternativen Ausgestaltung kann vorgesehen sein, dass die Integritätsprüfung für die Datenauslese-Applikation unabhängig von einem tatsächlichen Zugriff der externen Datenauslesevorrichtung 7 in dem Blutzuckermessgerät 1 aufgeführt wird, beispielsweise bei einem Gerätestart.

Die gespeicherte digitale Prüfsignatur wird mittels des Firmwaremoduls 3 auf den aktuellen Stand gebracht, wenn zum Beispiel eine neue Version der Datenauslese-Applikation installiert wird. Andererseits ermöglicht die vorgesehene Trennung von Signaturspeicher 4 einerseits und Firmwaremodul 3 andererseits eine Aktualisierung oder einen Austausch der Firmware, ohne dass hiervon die Speicherung der digitalen Prüfsignatur(en) betroffen ist. Auch die Datenauslese-Applikation kann unabhängig von der Firmware aktualisiert oder geändert werden. Im Anschluss hieran wird auch die in dem Signaturspeicher 4 gespeicherte digitale Prüf signatur aktualisiert, sodass für zukünftige Integritätsprüfungen die korrekte Prüfsignatur gespeichert ist.

## Patentansprüche

1. Blutzuckermessgerät (1), mit:
- einer Messeinrichtung (2) zum Bestimmen von Blutzuckermesswerten,
- einem Messwertspeicher (5), in welchem die Blutzuckermesswerte gespeichert sind,
- einer Datenschnittstelle (6), die für einen Datenaustausch mit einer externen Datenauslesevorrichtung (7) konfiguriert ist, und
- einem Datenfilesystem (10), welches eine Datenauslese-Applikation umfasst, auf die die externe Datenauslesevorrichtung (7) für ein Auslesen der gespeicherten Blutzuckermesswerte zugreifen kann,
wobei das Datenfilesystem (10) weiterhin eine Prüfapplikation umfasst, die konfiguriert ist, eine Integritätsprüfung für die Datenauslese-Applikation auszuführen, indem eine aktuelle digitale Signatur für die Datenauslese-Applikation gebildet und mit einer in dem Datenfilesystem (10) bereitgestellten digitalen Prüfsignatur verglichen wird.

2. Blutzuckermessgerät nach Anspruch 1, **gekennzeichnet durch** einen nichtflüchtigen Signaturspeicher (4), in welchem die digitale Prüfsignatur gespeichert ist.

3. Blutzuckermessgerät nach Anspruch 2, **gekennzeichnet durch** ein Firmwaremodul (3), welches konfiguriert ist, die digitale Prüfsignatur aus dem nichtflüchtigen Signaturspeicher auszulesen und an das Datenfilesystem zu übergeben.

4. Blutzuckermessgerät nach mindestens einem der vorangehenden Ansprüche, **dadurch gekennzeichnet, dass** die Prüfapplikation als eine Unterapplikation der Datenauslese-Applikation gebildet ist, derart, dass de Unterapplikation nach einem Aufrufen der Datenauslese-Applikation durch diese gestartet wird.

5. Blutzuckermessgerät nach mindestens einem der vorangehenden Ansprüche, **dadurch gekennzeichnet, dass** die Datenauslese-Applikation und die Prüfapplikation jeweils als eine Skriptapplikation gebildet sind.

6. Blutzuckermessgerät nach mindestens einem der vorangehenden Ansprüche, **dadurch gekennzeichnet, dass** die digitale Prüfsignatur in dem Datenfilesystem (10) in einer Konfigurationsdatei bereitgestellt ist.

7. Blutzuckermessgerät nach mindestens einem der vorangehenden Ansprüche, **dadurch gekennzeichnet, dass** die Datenauslese-Applikation konfiguriert ist, für einzelne Blutzuckermesswerte und / oder eine Gruppen von Blutzuckermesswerten eine Messwert-Integritätsprüfung auszuführen, indem eine digitale Messwert-Signatur gebildet und mit einer digitalen Messwert-Prüfsignatur verglichen wird.

8. Blutzuckermessgerät nach mindestens einem der vorangehenden Ansprüche, **dadurch gekennzeichnet, dass** die Datenauslese-Applikation und / oder die Prüfapplikation konfiguriert sind, ein Herunterladen der Blutzuckermesswerte auf die externe Datenauslesevorrichtung (7) und / oder ein Anzeigen herunter geladener Blutzuckermesswerte zu unterbinden, wenn die Prüfapplikation ergibt, dass die aktuelle digitale Signatur und die digitale Prüfsignatur in unzulässiger Weise nicht übereinstimmen.

9. Verfahren zum Auslesen von Blutzuckermesswerten von einem Blutzuckermessgerät (1), wobei das Verfahren die folgenden Schritte umfasst:
- Ausführen einer in einem Datenfilesystem (10) des Blutzuckermessgerätes (1) gebildeten Prüfapplikation, mittels der eine Integritätsprüfung für eine ebenfalls in dem Datenfilesystem (10) gebildete Datenauslese-Applikation ausgeführt wird, derart, dass eine aktuelle digitale Signatur für die Datenauslese-Applikation gebildet und mit einer in dem Datenfilesystem (10) bereitgestellten digitalen Prüfsignatur verglichen wird, und
- Unterbinden einer Übertragung von Blutzuckermesswerten von dem Blutzuckermessgerät (1) auf die externe Datenauslesevorrichtung (7) im Rahmen der Datenauslese-Applikation und / oder einer Anzeige übertragener Blutzuckermesswerte auf einer externen Anzeigeeinrichtung, wenn die Prüfapplikation ergibt, dass die aktuelle digitale Signatur und die digitale Prüfsignatur in unzulässiger Weise nicht übereinstimmen.
